# EUROPEAN PATENT APPLICATION

(11) **EP 1 906 167 A2**
(43) Date of publication of application: **02.04.2008**
(21) Application number: 07018938.6
(22) Date of filing: 26.09.2007
(51) Int. Cl.: G01N 1/28, G01N 33/49, B01L 3/00

(54) **Blood plasma collecting method and tool, and simplified blood testing method and tool**

(30) Priority: 27.09.2006 JP 2006263486; 29.09.2006 JP 2006269529
(71) Applicant: FUJIFILM Corporation, Minato-ku Tokyo (JP)
(72) Inventor: Takahashi, Kazunori, Ashigara-Kami-Gun Kanagawa 258-0022 (JP); Fujiwara, Takayuki, Minami-Ashigara-shi Kanagawa 250-0123 (JP)
(74) Representative: HOFFMANN EITLE

(57) **Abstract**

Plasma and corpuscles can be separated from each other requiring only a small quantity of blood in a short period of time easily and yet accurately with a small, simple-structured tool to enable the plasma to be collected without needing the use of any chemical such as a separation assisting agent. A plasma collecting tool (10) comprises a container (12) having a micro space (14) of an extremely thin rectangular shape measuring 50 µm to 2 mm in thickness, and a blood filling port (20) and a plasma collecting port (22) both communicating with the micro space (14); a syringe (16) which fills the micro space (14) of the container (12) with blood (A) via the blood filling port (20); an inclining device (19) which inclines the container (12) to achieve a prescribed angle of inclination relative to the vertical direction; and a syringe pump (18) which collects plasma (B) by sucking the same through the plasma collecting port (22).

## Description

### BACKGROUND OF THE INVENTION

### Field of the Invention

The present invention relates to a blood plasma collecting method and tool and simplified blood testing method and tool, and more particularly to a technique of efficiently collecting only blood plasma by separating blood plasma and blood corpuscles in blood in a short period of time as pretreatment for the blood test, and a technique of carrying out the process from blood plasma separation to the blood test as such in a simplified way with a single tool.

### Description of the Related Art

In performing a blood test, a few milliliter samples of blood are collected into vacuum blood collection pipes, each for one item of measurement or another, and the vacuum blood collection pipes are set to various automatic analyzers. Automatic analyzers are large apparatuses, which many hospitals cannot afford to equip themselves with, and these hospitals would entrust analyses to specialized laboratories. As a result, the doctors and patients have to wait long until they are told of the test results. If a test is urgently needed, the patient has to go directly to a medical facility equipped for blood testing, and if the patient is very old or seriously ill, the test itself will impose a heavy physical burden. If facilities are available for simpler and more frequent blood testing, prevention of diseases can be strengthened, and potential patients can live more comfortably.

Incidentally, blood put to be tested is often optically analyzed, and the pigment held by hemoglobin contained in solids in blood, such as erythrocytes, is known to obstruct colorimetry. For this reason, in order to achieve highly accurate test findings, the use of only blood plasma (supposed to include serum as well), obtained by separation of corpuscles from blood, as the sample is required as pre-treatment for the blood test. To enable corpuscles to be separated and removed from blood and blood plasma alone to be readily collected not only in a large hospital or laboratory equipped with an elaborate plasma collecting apparatus but also on the spot where blood testing is required, it is important to make the apparatus as small as a simple testing tool and simplify the blood plasma collecting method. Further to alleviate the physical and psychological burdens of the patient whose blood is to be tested, the required quantity of blood for the test should be much reduced.

Because of these background circumstances, the blood plasma collecting method and apparatus are called on to meet the following requirements.
(1) A small tool should be made available to enable plasma to be collected easily and from only a small quantity of blood. In order to make the blood sampling procedure simple enough for anyone to perform anywhere, the volume of blood that can be sampled with a lancet should be limited to a few tens of microliters at most.
(2) As fresh blood degenerates overtime and becomes unsuitable for testing, the time taken to collect plasma, namely the time taken to separate blood into plasma and blood corpuscles, should be minimized.
(3) Chemicals such as separation assisting agent may adversely affect the accuracy of testing, and therefore should not be used.

Currently available blood plasma collecting methods or tools include the following.

Japanese Patent Application Laid-Open No. 2003-83958 describes a method by which blood plasma and corpuscles are centrifugally separated, and this method not only takes a long time to accomplish the separation but also involves the trouble transferring the plasma to the testing apparatus after it is separated from blood corpuscles. Japanese Patent Application Laid-Open No. 2006-52950, disclosing an enlarged and improved version of a centrifugal apparatus, proposes a method by which blood is guided into a microchip and put to a centrifuge in the state of being held in the microchip. This method has its own limit in reducing the hardware size because the apparatus indispensably needs a high speed revolving unit.

Japanese Patent Application Laid-Open No. 2003-270239 concerns a method of segregating blood plasma from corpuscles by membrane separation. Though this method requires only a very small quantity of blood from which to separate plasma, its practice entails a complex procedure of diluting the sampled blood and extracting the plasma to be tested out of a very small container. Furthermore, as the membrane itself has a water holding capability, not only part of the sample is susceptible to loss but also the membrane may become clogged or the sample hemolyzed because the blood has to be pressed into a filter.

By the method according to Japanese Patent Application Laid-Open No. 2000-171461, which involves neither centrifugation nor membrane separation, a cation exchange substance and an anion exchange substance are alternately stacked over the surface of a substrate onto which blood is to be introduced to electrify the substrate surface with captions thereby to electrically catch anion-electrified erythrocytes (part of blood corpuscles). This method, however, not only involves the problem of more complex coating but also may well electrostatically adsorb contents of the plasma.

The method disclosed by Japanese Patent Application Laid-Open No. 2005-292092 is another non-centrifugal, non-membrane separation method, by which a flocculant (separation assisting agent) is added into a minute quantity of blood to cause the generated floccules to precipitate in a channel structure thereby to separate the solid and liquid contents of the blood from each other. This method, however, involves not only such problems as the complex shape of the channel and a long time taken by the flocculation to begin, but also a fear that the flocculant may adversely affect the blood test.

Furthermore, it is the usual practice at present to have plasma separated from corpuscles to be blood tested elsewhere, but this is not only a waste of time but also a wasteful use of the sample. Therefore it is called to have the plasma test in the same place as the separation of corpuscles.

### SUMMARY OF THE INVENTION

As stated above, every available blood plasma collecting method or apparatus has its own advantages and disadvantages, but none satisfies the requirements of (1) through (3).

Furthermore, if not only the collection of plasma but also the sequence from plasma separation to actual blood testing can be easily and yet accurately accomplished with a single simplified testing tool as a pre-treatment to blood testing, there will be no need to collect plasma and perform the blood test in separate places. This would enable potential patients to take blood tests in a more carefree way and more frequently, and the prevention of diseases would be thereby facilitated.

An object of the present invention, attempted in view of these circumstances, is to provide a blood plasma collecting method by which blood plasma and corpuscles can be separated from each other easily but accurately in a short period of time with a small and simply structured tool requiring only a small quantity of blood, and plasma can be collected without needing the use of any chemical such as a separation assisting agent, and a tool therefor.

Another object of the invention is to provide a simplified blood testing method and tool permitting the whole sequence from plasma separation to actual blood testing with a single simplified testing tool easily and yet accurately.

According to a first aspect of the invention, there is provided a blood plasma collecting method to separate corpuscles from blood and collect plasma, comprising a filling step of filling with blood, out of a micro space which is at least one long space formed in the horizontal direction, measuring 10 µm to 1 mm in depth in the direction of gravity, and a collection channel which communicates with the micro space upward in the direction of gravity and is smaller than the micro space in equivalent diameter, the micro space; a solid/liquid separation step of subjecting the blood to solid/liquid separation by gravity into plasma and corpuscles by allowing the blood in the micro space to stand still for a prescribed length of time; and a collection step of collecting the plasma, which is the supernatant liquid resulting from the solid/liquid separation, by sucking the collection channel.

According to the first aspect of the invention, the phenomenon of the natural precipitation of corpuscles in the blood by the action of gravity is cause to take place in the micro space as small as 10 µm to 1 mm deep in the direction of gravity and the filling step, solid/liquid separation step and collection step are accomplished with the equivalent diameter relationship between the micro space and the collection channel together with the angle (in the direction of gravity) relative to the micro space (the horizontal direction) being appropriately contrived, blood plasma and corpuscles can be separated from each other easily but accurately in a short period of time with a small and simply structured tool requiring only a small quantity of blood, and plasma can be collected without needing the use of any chemical such as a separation assisting agent.

Thus, at the filling step, out of the micro space which is at least one long space formed in the horizontal direction orthogonal to the direction of gravity, measuring only 10 µm to 1 mm in depth in the direction of gravity, and the collection channel which communicates with the micro space upward in the direction of gravity and is smaller than the micro space in equivalent diameter, the micro space is filled with blood. The reason why the depth of the micro space in the direction of gravity here is supposed to be 10 µm to 1 mm is that, since the size of corpuscles is about 8 µm, no sufficient precipitation depth can be secured unless the depth is 10 µm or more, and a precipitation distance of 1 mm is sufficient for separation of plasma from corpuscles. The preferable depth of the micro space is in a range of 20 µm to 50 µm, more preferably 20 µm to 200 µm, and particularly preferably from 20 µm to 100 µm.

At the solid/liquid separation step, the blood is subjected to solid/liquid separation by gravity into plasma and corpuscles by allowing the blood in the micro space to stand still for a prescribed length of time. Separation of plasma and corpuscles from each other by utilizing the micro space whose distance in the direction of gravity is extremely short, separation requiring only a small quantity of blood is made possible in a very short period of time. The precipitation velocity of corpuscles is about 3 µm/second with flocculation disregarded, and supposing that the depth of the micro space in the direction of gravity is 500 µm, the precipitation will be completed in less than five minutes. In order to shorten the time required for separation of plasma from corpuscles in this case, it is essential that only the micro space is filled with blood but blood does not reach the collection channel. If the collection channel is also filled with blood, the effective distance of the precipitation of corpuscles will be extended, and separation will take a longer time.

According to the invention, since the equivalent diameter of the collection channel is set shorter than that of the micro space, it can be made difficult for the blood to flow from the micro space into the collection channel. While the equivalent diameter of the collection channel is determined by the relationship with the surface tension of blood, preferably it may be half of the equivalent diameter of the micro space or even smaller.

Next, after the blood in the micro space has been allowed to stand still for a prescribed length of time, the collection channel is sucked to collect the plasma, which is the supernatant liquid resulting from the solid/liquid separation. What is essential at this collection step is that, as stated above, the collection channel is set smaller than that of the micro space in equivalent diameter, and the collection channel is erected upward in the direction of gravity relative to the horizontal micro space. In this way, by adjusting the sucking force to work on the collection channel appropriately, the precipitated corpuscles can be prevented from whirling up when the plasma is collected. Therefore, the corpuscles having once precipitated at the solid/liquid separation step can be prevented from becoming mixed again with the plasma collected at the collecting step.

According to the second aspect of the invention, which is a version of the first aspect, at the filling step blood is injected into a liquid reservoir formed at one lengthwise end of the micro space; and blood in the liquid reservoir is so filled into the micro space as not to reach the collection channel by sucking air in the micro space through an air escape formed at the other lengthwise end of the micro space or pressurizing the liquid reservoir in a state in which the collection channel is sealed or pressurized.

The second aspect concerns a preferable method of preventing the blood to fill the micro space from reaching the collection channel. First, blood is injected into the liquid reservoir formed at one lengthwise end of the micro space. In this state, with the collection channel being sealed or pressurized, the blood in the liquid reservoir is pressed into the micro space either by sucking air in the micro space little by little through the air escape formed at the other lengthwise end of the micro space or pressurizing the liquid reservoir.

In this way, the blood to fill the micro space can be fed without reaching the collection channel. Incidentally, the preferable pressure (differential pressure) to be applied to the collection channel is 0.05 to 50 KPa, a more preferable pressure range being 0.5 to 5 KPa. It is preferable to keep the sealed or pressurized state to be maintained until the precipitation of blood corpuscles in the micro space is completed.

According to the third aspect of the invention, which is a version of the second aspect, the micro space is treated for hydrophilicity and the collection channel is treated for hydrophobicity.

The third aspect concerns another method of preventing the blood to fill the micro space from reaching the collection channel. It is difficult for hydrophilic blood to flow into the collection channel treated for hydrophobicity.

According to the fourth aspect of the invention, which is a version of any one of the first through third aspects, at the solid/liquid separation step the temperature of the blood is kept in a range of 11 to 40°C.

While blood is highly viscous and its corpuscles are difficult to precipitate, the viscosity of the blood can be reduced to accelerate the precipitation of corpuscles by keeping the blood temperature in the range of 11 to 40°C. Incidentally, a temperature above 40°C would deteriorate the quality of blood, while a temperature below 11°C would not be so effective in accelerating the precipitation.

According to the fifth aspect of the invention, which is a version of any one of the first through fourth aspects, at the solid/liquid separation step the prescribed length of time for allowing the blood to stand still is within a range of three to six minutes.

The fifth aspect specifically sets forth the time taken to separate the plasma and corpuscles at the solid/liquid separation step, and supposes separation in the very small micro space whose depth in the direction of gravity is 10 µm to 1 mm. Therefore, at least three minutes would be sufficient for separating plasma from corpuscles, and even with a safety margin, six minutes would be long enough.

According to the sixth aspect of the invention, which is a version of any one of the first through fifth aspects, at the collection step plasma is sucked in a state in which the collection channel communicates with elsewhere than any lengthwise end of the micro space.

The sixth aspect represents a contrivance to accelerate the suction of plasma (supernatant) without causing the corpuscles having precipitated at the bottom of the micro space to whirl up. Thus, by sucking plasma in a state in which the collection channel communicates with elsewhere than any lengthwise end of the micro space, the suction of plasma can be accelerated without causing the corpuscles having precipitated to whirl up.

The "state in which the collection channel communicates with elsewhere than any lengthwise end of the micro space" in this context means a state in which the collection channel erects from a position near the center of the long micro space in an inverted T shape, for instance. The "state in which the collection channel communicates with a lengthwise end of the micro space" may also mean a state in which the collection channel erects from one end position of the long micro space in something like an L shape.

According to the seventh aspect of the invention, which is a version of any one of the first through sixth aspects, at the collection step the plasma as the supernatant liquid of the micro space is so sucked into the collection channel at a flow rate of 1 mm/second or below.

The seventh aspect concerns prescription of the transfer speed of the plasma at the time of its recover to prevent precipitated corpuscles from whirling up. Thus, by causing the plasma in the micro space to flow into the collection channel at a flow rate of 1 mm/second or below, the plasma can be collected while preventing the precipitated corpuscles from whirling up.

In order to achieve the foregoing objects of the invention, according to its eighth aspect, there is provided a blood plasma collecting tool to collect separate corpuscles from blood and collect plasma, comprising a tool body within whose substrate a micro space which is at least one long space formed in the horizontal direction, measuring 10 µm to 1 mm in depth in the direction of gravity, a collection channel which communicates with the micro space upward in the direction of gravity and is smaller than the micro space in equivalent diameter, a liquid reservoir which, formed at one lengthwise end of the micro space, communicates with the atmosphere, an air escape which, formed at the other lengthwise end of the micro space, communicates with the atmosphere, and a sucking part which, formed at the tip of the collection channel, communicates with the atmosphere are formed; a filling device which fills the inside of the micro space with blood via the liquid reservoir; and a sucking device connected to the sucking part.

The eighth aspect concerns the configuration of the invention as hardware, and according to this aspect blood plasma and corpuscles can be separated from each other easily but accurately in a short period of time with a small and simply structured tool requiring only a small quantity of blood, and plasma can be collected without needing the use of any chemical such as a separation assisting agent.

According to the ninth aspect of the invention, which is a version of any one of the eighth aspect, the collection channel is connected to the central position lengthwise of the micro space.

Since the collection channel is connected to the central position lengthwise of the micro space according to the ninth aspect, the micro space and the collection channel constitute an inverted T shape as mentioned above. This arrangement enables the suction of plasma to be accelerated without causing the corpuscles having precipitated to whirl up.

According to the tenth aspect of the invention, which is a version of any one of the eighth or ninth aspect, the width of the long micro space is in a range of 50 µm to 5 mm and the length of the same in the horizontal direction is from 1 to 20 cm.

The tenth aspect prescribed the preferable width and length ranges in the horizontal direction in addition to the depth of the micro space in the direction of gravity. Thus, while the depth of the micro space in the direction of gravity is 10 µm to 1 mm as stated above, its preferable width and length are respectively 50 µm to 5 mm and 1 to 20 cm. Although it is not preferable for the depth of micro space in the direction of gravity to be dramatically varied from the range stated above, the width and length in the horizontal direction can be appropriately altered to match the required quantity of plasma for the blood test. Further, a sufficient micro space length can be secured by radially arranging a plurality of micro spaces centering on the collection channel.

According to the eleventh aspect of the invention, which is a version of any one of the eighth through tenth aspects, the inside surface of the micro space is treated for hydrophilicity and the inside surface of the collection channel is treated for hydrophobicity.

The eleventh aspect enables the blood to fill the micro space to be prevented from reaching the inside of the collection channel.

According to the twelfth aspect of the invention, which is a version of any one of the eighth through eleventh aspects, the tool body is transparent.

The twelfth aspect enables the state of separation between the plasma and the corpuscles to be visually recognized and accordingly the user to readily know when the separation has ended.

According to the thirteenth aspect of the invention, which is a version of any one of the eighth through twelfth aspects, the material of the tool body is any of polystyrene resin, acryl resin, PDMS resin, polycarbonate resin, PET resin, quartz glass, Pyrex® and silicon.

The thirteenth aspect specifically refers preferable materials for the tool body according to the invention.

According to the fourteenth aspect of the invention, which is a version of any one of the eighth through thirteenth aspects, a plurality of the collection channels are provided at intervals in the lengthwise direction of the micro space.

Since a plurality of the collection channels are provided at intervals in the lengthwise direction of the micro space according to the fourteenth aspect, the time taken to collect plasma can be shortened.

In order to achieve the foregoing objects of the invention, according to its fifteenth aspect, there is provided a blood plasma collecting method to separate corpuscles from blood and collect plasma, comprising a filling step of filling with blood a container having a micro space of an extremely thin rectangular shape measuring 50 µm to 2 mm in thickness; an inclining step of inclining the container to an inclination angle of 30 to 60° to the vertical direction before or after the filling step; a solid/liquid separation step of subjecting blood to solid/liquid separation by gravity into plasma and corpuscles by allowing the blood to stand still for a prescribed length of time in the inclined container; and a collection step of collecting the plasma having undergone solid/liquid separation by sucking from the container

"Inclining the container [relative] to the vertical direction" means both inclining downward the container from a state in which it is stood in the vertical direction (the direction of gravity) toward the thickness direction of the micro space and inclining upward the container from a state in which it is laid to the thickness direction of the micro space toward the thickness direction of the micro space. The container in this context means one whose length is much greater than its width, such as a test tube.

The fifteenth aspect concerns enabling plasma and blood corpuscles to be separated from each other in a very short period of time, though only relying on natural precipitation by gravity, by applying the Boycott effect in solid/liquid separation to the container having a micro space in an extremely thin rectangular shape measuring 50 µm to 2 mm in thickness.

Thus at the filling step, the container having a micro space in an extremely thin rectangular shape measuring 50 µm to 2 mm in thickness is filled with blood. The reason why the thickness of the micro space here is supposed to be 50 µm to 2 mm is that, when the container is inclined by 30 to 60°, a thickness of less than 50 µm would make it difficult for the Boycott effect to exert itself, while a thickness greater than 2 mm would entail too long a precipitation distance for the corpuscles, which could not precipitate in a sufficiently short period of time. Therefore, since the thickness of the micro space and the angle of inclination of the container are in a mutually limiting relationship, it is preferable to conduct experiments in advance and find an appropriate combination of thickness and inclination angle in the range of thickness and that of inclination angle stated above.

Then at the solid/liquid separation step, blood is allowed to stand still for a prescribed length of time in the micro space to separate the blood into plasma and corpuscles by gravity. It may be worth mention here that the behavior of corpuscles within the micro space varies with the standing direction of the container relative to the direction of gravity. Corpuscles in a vertically erected container move in the direction of gravity, namely in the vertical direction. At the same time, plasma moves in the reverse direction to bring corpuscles and plasma into head-on collision, making it difficult for the corpuscles to precipitate.

Unlike that, at the solid/liquid separation step according to the invention, blood corpuscles precipitate in the direction of gravity in the micro space in the inclined container, while plasma rises along the upper face of the upper part of the micro space in the thickness direction. This prevents the precipitating direction of the corpuscles and the rising direction of the plasma from being exactly reverse to each other. As a result, it is made difficult for the two streams to interfere with each other, resulting in the separation of plasma and corpuscles in a short period of time. Moreover, as the space size is in the order of micrometers, the effective precipitation distance of blood corpuscles can be significantly reduced by inclining the space.

By applying the Boycott effect to the micro space in this way, plasma and corpuscles can be separated in a very short period of time requiring only a small quantity of blood. Though the collection is carried out after the separation, the plasma having undergone solid/liquid separation is collected by sucking from the container. This enables blood plasma and corpuscles to be separated from each other easily but accurately in a short period of time with a small and simply structured tool requiring only a small quantity of blood, and plasma can be collected without needing the use of any chemical such as a separation assisting agent.

A more preferable thickness range of the micro space is from 100 µm to 500 µm. A particularly preferable inclination angle is 45°.

According to the sixteenth aspect of the invention, which is a version of the fifteenth aspect, at the solid/liquid separation step the temperature of the blood is kept in a range of 11 to 40°C.

While blood is highly viscous and its corpuscles are difficult to precipitate, the viscosity of the blood can be reduced to accelerate the precipitation of corpuscles by keeping the blood temperature in the range of 11 to 40°C. Incidentally, a temperature above 40°C would deteriorate the quality of blood, while a temperature below 11°C would not be so effective in accelerating the precipitation.

According to the seventeenth aspect of the invention, which is a version of the fifteenth or sixteenth aspect, at the solid/liquid separation step the prescribed length of time for allowing the blood to stand still is within a range of three to five minutes.

The seventeenth aspect specifically sets forth the length of time for separation of plasma and corpuscles from each other at the solid/liquid separation step, and supposes that at least three minutes would be sufficient for separating plasma from corpuscles, and even with a safety margin, five minutes would be long enough.

In order to achieve the foregoing objects of the invention, according to its eighteenth aspect, there is provided a simplified blood testing method for testing blood in a simplified way, comprising a filling step of filling with blood a container having a micro space of an extremely thin rectangular shape measuring 50 µm to 2 mm in thickness; an inclining step of inclining the container to an inclination angle of 30 to 60° to the vertical direction before or after the filling step; a solid/liquid separation step of subjecting blood in the inclined container to solid/liquid separation by gravity into plasma and corpuscles by allowing the blood to stand still for a prescribed length of time; and a testing step of bringing a substance to be tested in the plasma having undergone the solid/liquid separation into contact with a testing reagent fastened in advance to the upper part of the upper face of the opposing faces of the micro space in the thickness direction in the inclined state, and thereby conducting a test.

The eighteenth aspect utilizes the advantage that applying the Boycott effect to the micro space can not only reduce the time taken to separate plasma from corpuscles but also enable the upward stream of the plasma to flow along the upper face of the micro space. Thus, as the advance fastening the testing reagent in advance to the upper part of the upper face of the micro space contributes to the efficiency of contact between the substance to be tested in (a content of) the plasma and the testing reagent, and accordingly the sequence from plasma separation to blood testing can be accomplished in a consistent accurate process in a simple space such as the micro space.

According to the nineteenth aspect of the invention, which is a version of the eighteenth aspect, the substance to be tested in the plasma is an antibody, the testing reagent is an antibody, and the testing is accomplished by causing an antigen-antibody reaction to take place at the testing step.

The nineteenth aspect represents a preferable mode of implementing the invention, in which blood testing by an antigen-antibody reaction can be easily and yet accurately carried out.

In the simplified blood testing method according to the invention as well, it is preferable to keep the blood temperature in the range of 11 to 40°C at the solid/liquid separation step. Further at the solid/liquid separation step, it is preferable to keep the prescribed length of time for allowing the blood to stand still within a range of three to five minutes.

In order to achieve the foregoing objects of the invention, according to its twentieth aspect, there is provided a plasma collecting tool which separates corpuscles from blood and collects plasma, comprising a container having inside a micro space of an extremely thin rectangular shape measuring 50 µm to 2 mm in thickness, and a blood filling port and a plasma collecting port both communicating with the micro space; a filling device which fills the micro space of the container with blood via the blood filling port; an inclining device which inclines the container to achieve a prescribed angle of inclination relative to the vertical direction; and a sucking device which collects plasma by sucking the same through the plasma collecting port.

The twentieth aspect concerns the configuration of the invention as hardware, and according to this aspect blood plasma and corpuscles can be separated from each other easily but accurately in a short period of time with a small and simply structured tool requiring only a small quantity of blood, and plasma can be collected without needing the use of any chemical such as a separation assisting agent.

According to the twenty-first aspect of the invention, which is a version of the twentieth aspect, the angle of inclination is in a range of 30 to 60°.

The twenty-first aspect represents a suitable angle of inclination of the container for use where the Boycott effect is applied to the micro space, of which the preferable angle of inclination is in a range of 30 to 60°, a particularly preferable angle of inclination being 45°.

According to the twenty-second aspect of the invention, which is a version of the twentieth or twenty-first aspect, an aspect ratio represented by L/D, where D is the thickness and L is the length of the micro space, is within a range of 5 to 50.

The twenty-second aspect sets forth a suitable aspect ratio of the length L to the thickness D of the micro space. The precipitation velocity of blood corpuscles tends to be proportional to the aspect ratio where it is within a range of 5 to 50.

According to the twenty-third aspect of the invention, which is a version of any one of the twentieth through twenty-second aspects, the container is transparent.

A transparent container not only enables the time of the end of separation between the plasma and the corpuscles to be visually recognized but also enables the precipitation of blood corpuscles and a state of upward flow of the plasma to be visually recognized and accordingly the user can adjust the container to an appropriate angle of inclination.

According to the twenty-fourth aspect of the invention, which is a version of any one of the twentieth through twenty-third aspects, the material of the container is any out of resins, quartz glass and Pyrex®.

The twenty-fourth aspect sets forth particularly suitable materials for the container.

According to the twenty-fifth aspect of the invention, which is a version of any one of the twentieth through twenty-fourth aspects, a heating device for heating the container is further provided.

While blood is highly viscous and its corpuscles are difficult to precipitate, the viscosity of the blood can be reduced to accelerate the precipitation of corpuscles by heating the blood with a heating device (preferably to 11 to 40°C). Incidentally, a temperature above 40°C would deteriorate the quality of blood, while a temperature below 11 °C would not be so effective in accelerating the precipitation.

In order to achieve the foregoing objects of the invention, according to its twenty-sixth aspect, there is provided a simplified blood testing tool for simplified testing of blood, comprising a container having inside a micro space of an extremely thin rectangular shape measuring 50 µm to 2 mm in thickness, wherein a testing reagent is fastened in advance to one of the opposite faces of the micro space opposing in the thickness direction, and a blood filling port and an air escape both communicating with the micro space; a filling device which fills the micro space of the container with blood via the blood filling port; and an inclining device which inclines the container to achieve a prescribed angle of inclination relative to the vertical direction and to position upward the one face to which the testing reagent is fastened.

The twenty-sixth aspect concerns the configuration of the invention as hardware, and the use of this simplified blood testing tool enables the sequence from plasma separation to blood testing to be accomplished with a single simplified testing tool easily but accurately.

According to the twenty-seventh aspect of the invention, which is a version of the twenty-sixth aspect, the testing reagent is an antibody, and an antigen in the plasma, which is a content of the blood, is tested.

Here is shown a preferable mode of implementing the invention, in which blood testing by antigen-antibody reaction can be carried out easily and yet accurately.

Further, in a simplified blood testing tool according to the invention, it is preferable for the container to have an inclination angle in a range of 30 to 60°. The aspect ratio represented by L/D, where L is the length and D is the thickness of the micro space may preferably be within a range of 5 to 50. It is also preferable for the container to be transparent, made of a transparent resin such as acryl, quartz glass, Pyrex® or the like. It is further preferable to provide a heating device to warm the container to keep the temperature of the blood to undergo solid/liquid separation in a range of 11 to 40°C. In the solid/liquid separation, it is preferable to keep the prescribed length of time for allowing the blood to stand still within a range of three to five minutes.

As described so far, by the blood plasma collecting method and tool according to the invention, blood plasma and corpuscles can be separated from each other easily but accurately in a short period of time with a small and simply structured tool requiring only a small quantity of blood, and plasma can be collected without needing the use of any chemical such as a separation assisting agent.

Further, the simplified blood testing method and tool according to the invention permits the whole sequence from plasma separation to actual blood testing with a single simplified testing tool easily and yet accurately.

### BRIEF DESCRIPTION OF THE DRAWINGS

Fig. 1 is a conceptual diagram of a plasma collecting tool, which is a first preferred embodiment of the present invention;
Fig. 2 illustrates the differentiation of surface treatment between a micro space and a collection channel;
Figs. 3A to 3C illustrate the procedure of a blood plasma collecting method in the first preferred embodiment of the invention;
Fig. 4 shows a perspective view of a stand on which the plasma collecting tool is to be erected;
Figs. 5A and 5B illustrate a case in which the micro space and the collection channel constitute an inverted T shape and another in which they constitute an L shape;
Fig. 6 is a conceptual diagram of a blood plasma collecting tool according to the invention in another mode, wherein a plurality of collection channels are provided;
Fig. 7 is a conceptual diagram of a plasma collecting tool, which is a second preferred embodiment of the invention;
Figs. 8A to 8C illustrate the procedure of a blood plasma collecting method in the second preferred embodiment of the invention;
Figs. 9A and 9B illustrate the Boycott effect; and
Fig. 10 is a conceptual diagram of one example of simplified blood testing tool according to the invention.

### DETAILED DESCRIPTION OF THE PREFERRED EMBODIMENTS

The blood plasma collecting method and tool, and the simplified blood testing method and tool which are preferred embodiments of the invention will be described in detail below with reference to the accompanying drawings.

### (Plasma Collecting Method and Tool in the First Embodiment)

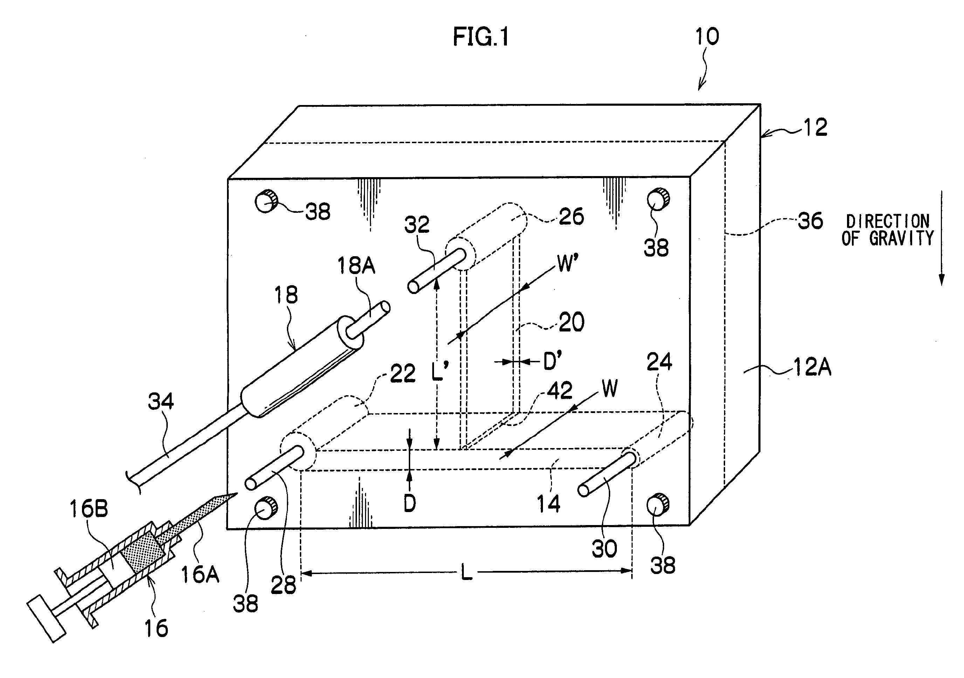

Fig. 1 is a conceptual diagram of one example of plasma collecting tool 10 in one embodiment of the invention.

As shown in Fig. 1, the plasma collecting tool 10 according to the invention mainly comprises a tool body 12, a filling device 16 for filling within blood a micro space 14 formed within the tool body 12, and a sucking device 18 for sucking plasma (supernatant liquid) separated from corpuscles in the micro space 14.

Within a board 12A constituting the tool body 12, there are the micro space 14 which is a space formed long in the horizontal direction (the direction orthogonal to the direction of gravity) and as shallow as 10 µm to 1 mm in the depth D in the direction of gravity, a collection channel 20 communicating upward with the micro space 14 in the direction of gravity and smaller than the micro space 14 in equivalent diameter, a liquid reservoir 22 formed at one end of the long micro space 14 and communicating with the atmosphere, an air escape 24 formed at the other end of the long micro space 14 and communicating with the atmosphere, and a sucking intake 26 formed at the tip of the collection channel 20 and communicating with the atmosphere. As is seen from Fig. 1, the collection channel 20 is formed in the upward direction of gravity relative to the micro space 14, and its communicating position is formed in the central part lengthwise of the micro space 14. This causes the micro space 14 and the collection channel 20 to form an inverted T shape.

The equivalent diameter here means the supposed diameter of a circle into which the section of the space or the channel is converted.

In prescribing the dimensions of the micro space 14 other than the depth D, it is preferable for the width W to be from 50 µm to 5 mm, more preferable from 100 µm to 1000 µm, and for the length L, to be from 1 to 20 cm, more preferable a length satisfying the requirement for the quantity of plasma needed for the blood test. The same can be said of the width W' of the collection channel 20 as of the micro space 14, and the preferable range for its length L' is 1 to 10 cm. It is preferable for the equivalent diameter of the collection channel 20 to be half that of the micro space 14 or less as stated above, and this relationship makes its preferable thickness D' to be half the thickness D of the micro space 14 or less. Further, it is preferable for the thickness D' of the collection channel 20 to be 1/4 of the micro space 14 or less.

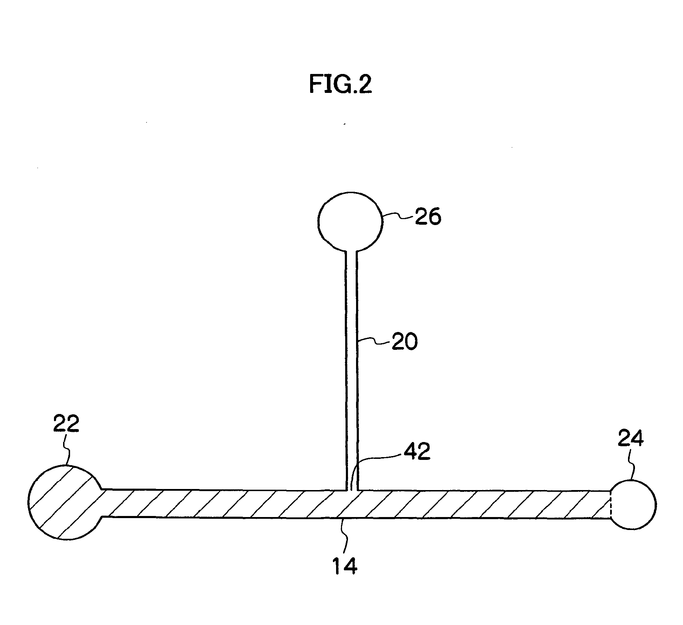

Further, as shown in Fig. 2, it is preferable for the inside surface of the micro space 14 to be treated for hydrophilicity and that of the collection channel 20 to be treated for hydrophobicity. Treatment for hydrophilicity or hydrophobicity in this context is not limited to hydrophilic or hydrophobic coating or lamination, but may also mean the choice of a hydrophilic or hydrophobic material. Suitable materials for the plasma collecting tool 10 include resins such as acryl, polyethylene terephthalate (PET) and polycarbonate, quartz, and glass such as Pyrex®. Therefore, since resins need no particular treatment to be hydrophobic, when the plasma collecting tool 10 is to be fabricated of a resin, it is advisable to apply an aqueous solution of polyethylene glycol resin (PEG) to the inside surface of the micro space 14 to make it hydrophilic. Conversely, the plasma collecting tool 10 is to be fabricated of an intrinsically hydrophilic material such as Pyrex® or some other glass, it is recommended to treat the inside surface of the collection channel 20 for hydrophobicity with a fluorine-based or hydrocarbon-based silane coupling agent. The glass, washed with a strong aqueous solution of alkali (e.g. potassium hydroxide of 5 mol/L) for one hour, can be treated for hydrophobicity with a solution prepared by dissolving a toluene solution of perfluorododecyl triethoxy silane (a product of Shin-Etsu Chemical) or octadecyl trichloro silane in an 89% aqueous solution of ethanol and adjusting its concentration to 1%.

For application of a hydrophilic or hydrophobic liquid, in the plasma collecting tool 10, the board 12A is formed of two plates, the lid and the body plate, a groove to serve as the micro space 14 and the collection channel is formed in the body plate, and the liquid is applied to the groove in drips from the nozzle of an ink jet device. It can as well be applied to the lit. It is preferable for the material of the lid, like that of the body plate, to be resin or glass, and this material is stuck to the board while being aligned. The sticking can be accomplished with a tacking agent or an adhesive, and the lid may preferably be made of glass where the material of the body plate is glass or silicon. The joining may preferably be accomplished by anodic bonding or pressure welding with hydrogen fluoride (hydrofluoric acid bonding).

Treatment of the micro space 14 for hydrophilicity in this way enables blood, which is hydrophilic, to smoothly flow into the micro space 14, but not as far as to the collection channel 20 which has been treated for hydrophobicity. Incidentally, filling with blood down to the collection channel 20 increases the substantial precipitating distance and extends the duration of separation between plasma and corpuscles. It is preferable for the liquid reservoir 22 to be treated for hydrophilicity as is the micro space 14 and for the air escape 24 and the sucking intake 26 to be treated for hydrophobicity. Thus, the hatched part in Fig. 2 is treated for hydrophilicity and the blank part, for hydrophobicity.

Further, as shown in Fig. 1, a filling pipe 28, an air vent 30 and a sucking pipe 32 are respectively connected to the liquid reservoir 22, the air escape 24 and the sucking intake 26.

As the filling device 16 for filling the micro space 14 with blood, a syringe 16 can be suitably used as shown in Fig. 1. Thus, the needle 16A of the syringe 16 holding blood inside is inserted into the filling pipe 28, and the piston 16B of the same is pressed in to fill the liquid reservoir 22 with the blood in the syringe 16.

As the sucking device 18 for collecting the separated plasma via the collection channel 20, a continuously fluid type syringe pump 18 also provided with a suction pressure control function can be suitably used as shown in Fig. 1. This enables the suction pressure and the suction flow rate to be controlled as desired. Thus, the tip outlet 18A of the syringe pump 18 is connected to the sucking pipe 32, and a collecting tube 34 is also connected to a blood testing apparatus (not shown). This enables the plasma (supernatant liquid) separated in the micro space 14 to be directly fed to the blood testing apparatus via the collection channel 20.

To fabricate within the board 12A the tool body 12 in which the micro space 14, the collection channel 20, the liquid reservoir 22, the air escape 24 and the sucking intake 26 are formed, micro machining techniques are applied. Examples of such micro machining techniques include the following. As stated above, the two plates including the lid and the body plate are positioned in the area represented by dotted lines 36 in Fig. 1, a half each of the micro space 14, the collection channel 20, the liquid reservoir 22, the air escape 24 and the sucking intake 26 being formed in the lid and the body plate, which are to be put together afterwards. It is preferable for the tool body 12 to be assembled by festinating at least the four corners of the board 12A with bolts 38.
(1) LIGA technique combining X-ray lithography and electroplating;
(2) High aspect ratio photolithography using EPON SU8;
(3) Mechanical micro cutting (micro drilling with a fast revolving drill whose diameter is in a micro order);
(4) High aspect ratio machining of silicon by deep RIE
(5) Hot embossing
(6) Stereolithography
(7) Laser machining
(8) Ion beam machining

There are a wide variety of board materials available for the tool body 12, from which one or another can be selected according to different requirements regarding resistance to heat, resistance to pressure, resistance to solvents and machining ease among other factors, and particularly preferable ones include polystyrene resin, acryl resin, PDMS resin, polycarbonate resin, PET resin, quartz glass, Pyrex^{®} and silicon. As will be described afterwards with reference to the plasma collecting method, it is preferable for the tool body 12 to be fabricated of a transparent material so that the state of separation between plasma and corpuscles can be visually recognized.

It is preferable for the tool body 12 to have a heating device (not shown). One of the conceivable heating devices is to build a heater structure made of a metal resistance line or polysilicon into the tool body 12. Where a heater structure made of a metal resistance line or polysilicon is to be used, this structure is used for positive heating, and cooling is accomplished by controlling the temperature by having a thermal cycle function by natural cooling. For temperature sensing, where a metal resistance line is to be used, the usual way is to build in another resistance line in advance, detect the temperature according to the variations in its resistance level. Where polysilicon is to be used, the usual practice is to detect the temperature with a thermoelectric couple. More recently, it has become possible to accomplish accurate temperature control of blood by building a temperature control function using a Peltier element into the tool body 12. At any rate, temperature control as such can be carried out either by a conventional technique or a novel one, typically a method using a Peltier element, and the optimal method can be selected by choosing a heating/cooling mechanism and a temperature sensing mechanism matching the material and other factors of the apparatus body and combining the selected mechanisms with an external control system.

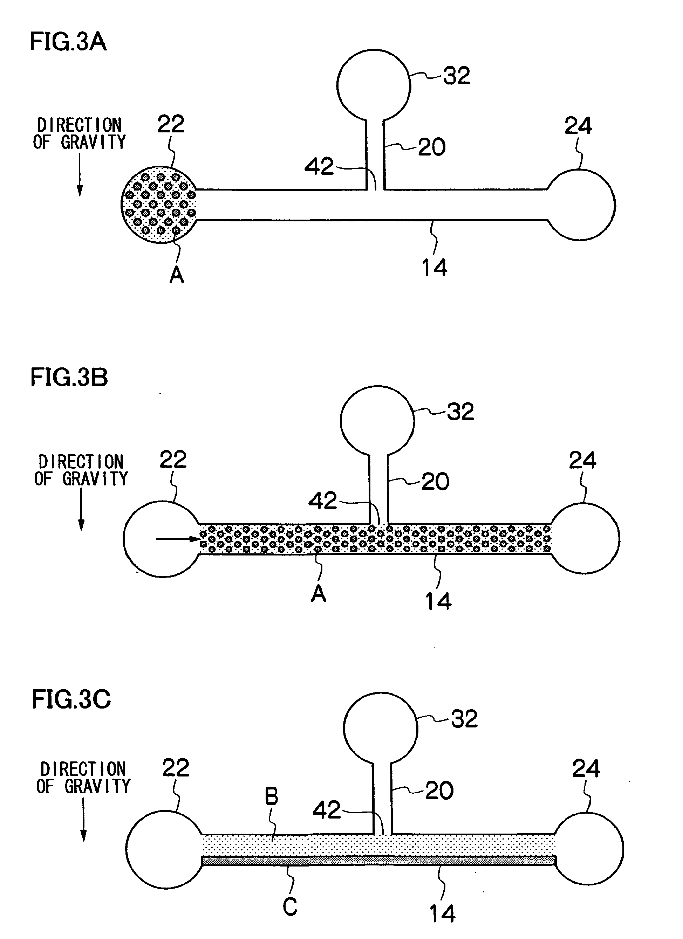

Next, the procedure of plasma collection according to the invention using the plasma collecting tool 10 configured as stated above will be described with reference to Fig. 1 and Figs. 3A to 3C.

The blood plasma collecting method according to the invention mainly comprises a filling step of filling the micro space 14 of the tool body 12 with blood A, a solid/liquid separation step of holding the blood A in the micro space 14 at rest for a prescribed period time and subjecting the blood A to solid/liquid separation by gravity into plasma B and corpuscles C, and a collection step of collecting the plasma B, which is the supernatant liquid, having undergone solid/liquid separation by sucking from the collection channel 20.

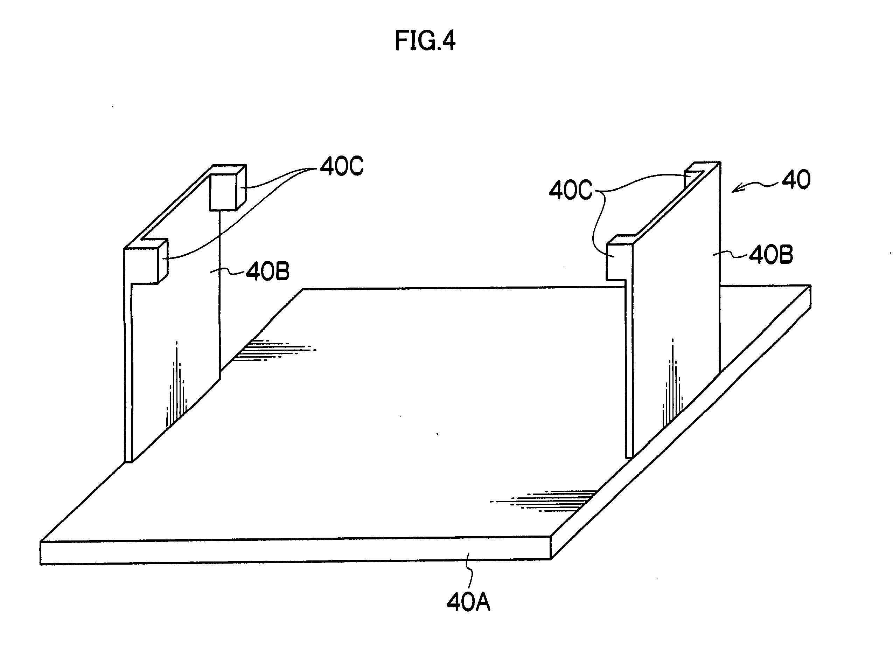

First, as shown in Fig. 1, the tool body 12 is so stood as to position the micro space 14 of the tool body 12 in a horizontal direction relative to gravity. In this case, it is advisable to use a stand 40 shown in Fig. 4, and snap the tool body 12 onto the claws 40C of a pair of side boards 40B stood on the two sides of the base 40A of this stand 40.

Next, the filling step of filling the micro space 14 with the blood A is implemented by using the syringe 16 shown in Fig. 1. At this filling step, as shown in Fig. 3A, the needle 16A of the syringe 16 holding the blood A inside is inserted into the filling pipe 28 to inject the blood A into the liquid reservoir 22. The syringe 16 from which the blood A has been injected is removed from the filling pipe 28 to keep the liquid reservoir 22 open to the atmosphere. It is preferable for the capacity of the liquid reservoir 22 to be equal to or greater than that of the micro space 14. Then in a state in which the collection channel 20 is intercepted from the atmosphere by sealing the tip of the sucking pipe 32 shown in Fig. 1 with a cap or the like, the pressure in the micro space 14 is reduced to cause the blood A in the liquid reservoir 22 to flow into the micro space 14 by inserting the needle of another syringe into the air vent 30 of the air escape 24 and pulling the piston part of the syringe.

Although the tip of the sucking pipe 32 is sealed with a cap or the like to intercept the collection channel 20 from the atmosphere according to the foregoing method, pressure may as well be applied to the collection channel 20 by fitting the tip of the sucking pipe 32 with a syringe pump. The preferable pressure (differential pressure) to be applied to the collection channel 20 is 0.05 to 50 KPa, a more preferable pressure range being 0.5 to 5 KPa. It is preferable to keep the sealed or pressurized state to be maintained until the precipitation of blood corpuscles in the micro space is completed. Further, though the blood A in the liquid reservoir 22 is caused to flow into the micro space 14 by reducing the pressure in the micro space 14 according to the method described above, pressure may as well be applied into the liquid reservoir 22 to press the blood A in the liquid reservoir 22 into the micro space 14.

This can cause the blood A in the liquid reservoir 22 to fill only the micro space 14 as shown in Fig. 3B and to be prevented from reaching the collection channel 20. In this case, it is preferable for the equivalent diameter of the collection channel 20 to be half that of the micro space 14 or even smaller. Thinning the collection channel 20 in this way enables the relationship with the surface tension of the blood A to make it difficult for the blood A to flow into the collection channel 20. It is further preferable for the inside surface of the micro space 14 to be treated for hydrophilicity and that of the collection channel 20, for hydrophobicity. Treating for hydrophilicity and hydrophobicity in this way serves not only to facilitate the flow of the hydrophilic blood A, when it is to fill the micro space 14, from the liquid reservoir 22 into the micro space 14 but also to obstruct it into the hydrophobic collection channel 20. Therefore, it is made even easier for the blood A to fill only the micro space 14.

Next to be implemented is the solid/liquid separation step of subjecting the blood A to solid/liquid separation by gravity into the plasma B and the corpuscles C by allowing the blood A to stand still in the micro space 14 for three to five minutes. At this solid/liquid separation, it is preferable to keep the tool body 12 at 11 to 40°C and thereby to reduce the viscosity of the blood A. This causes, as shown in Fig. 3C, the corpuscles C in the blood to the bottom of the micro space 14 in a short period of time while the plasma B stays in the upper part of the micro space 14 as the supernatant liquid.

Next, the syringe pump 18 is connected to the sucking pipe 32 of the collection channel 20 as shown in Fig. 1 to implement the collection step at which only the plasma B as the supernatant liquid is collected. Thus, it is sufficient for the sucking force of the syringe pump 18 to be able to break the surface tension of the plasma B on the boundary 42 between the micro space 14 and the collection channel 20 and to break the force of the collection channel 20 by its hydrophobicity to prevent the plasma from flowing in. It is preferable, for instance, for the sucking force to exert itself to so cause the plasma B in the micro space 14 as to flow into the collection channel 20 at a rate of not more than 1 mm/second. Too great a sucking force might whirl up the corpuscles C which have precipitated to become mixed with the plasma B to be collected. The collected plasma B is fed to the blood testing apparatus via the collecting tube 34 of the syringe pump 18.

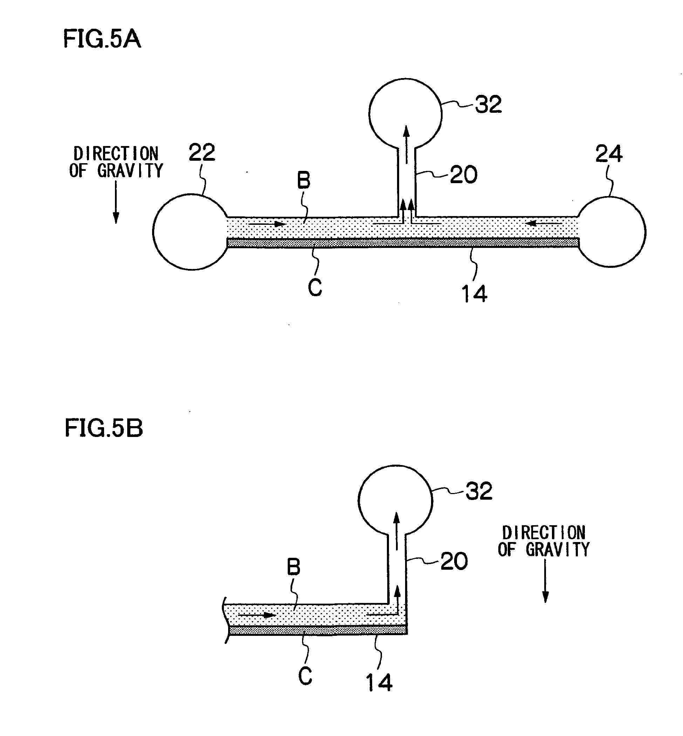

At this collection step, by so arranging the micro space 14 and the collection channel 20 as to form an inverted T shape as shown in Fig. 5A, the flow rate of the plasma B into the collection channel 20 can be increased without whirling up the precipitated corpuscles C more easily than the L shaped arrangement shown in Fig. 5B. Therefore, the total length of collecting time from the filling step through the collection step can be shortened.

This embodiment as described above is supposed to have only one micro space 14, but a plurality of micro spaces 14 may be provided as well. For instance by radially arranging a plurality of micro spaces 14 centering on the collection channel 20, a sufficient length can be secured for the whole micro spaces 14.

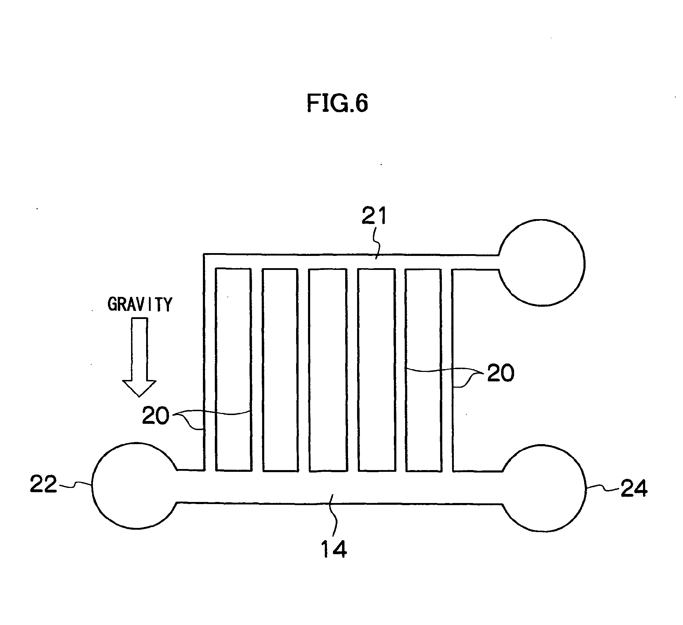

Also, though this embodiment of the invention as described above is supposed to have only one collection channel 20, as shown in Fig. 6 a plurality of (six in Fig. 6) collection channels 20 may be arranged at intervals in the lengthwise direction of the micro space 14, and the plurality of collection channels 20 joined into a single combined pipe 21 to gather the plasma into a collected liquid reservoir 23.

### (Plasma Collecting Method and Tool in the Second Embodiment)

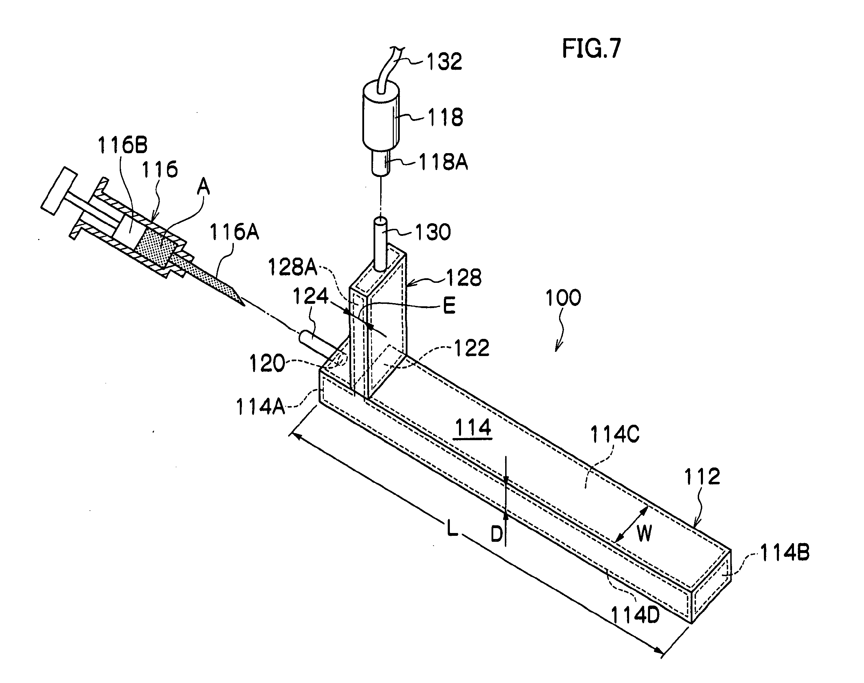

Fig. 7 is a conceptual diagram of a plasma collecting tool 100, which is a second preferred embodiment of the invention.

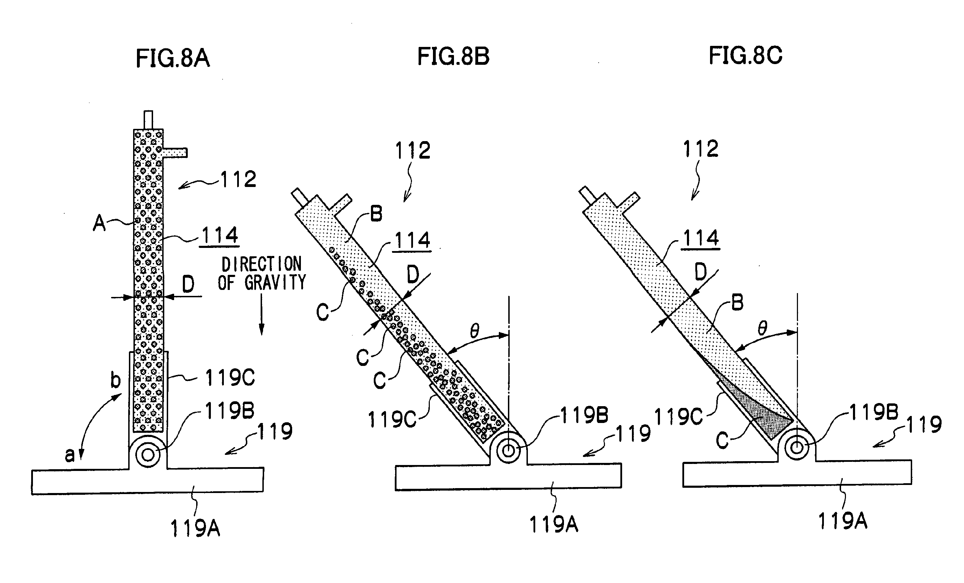

As shown in Fig. 7, the plasma collecting tool 100 according to the invention mainly comprises a container 112, a filling device 116 for filling a micro space 114 formed in the container 112 with blood, a sucking device 118 for sucking plasma (supernatant liquid) out of plasma and corpuscles into which blood has been separated in the micro space 114, and an inclining device 119 for inclining the container 112 (see Figs. 8A to 8C).

Within the container 112, the micro space 114 of an extremely thin rectangular shape measuring 50 µm to 2 mm in thickness D is formed. The preferable range of the thickness D is from 100 µm to 500 µm. It is preferable to set the length L of the container 112 in relation to the thickness D, to keep an aspect ratio represented by L/D, wherein D is the thickness and L is the length of the micro space 114, within a range of 5 to 50. For instance where the thickness D of the micro space 114 is 50 µm, the length L is kept between 250 µm and 2.5 mm or, where the thickness D of the micro space 114 is 2 mm, the length L is kept between 10 mm and 100 mm. Therefore, the size of the micro space 114 can be set to meet the prerequisites according to the quantity of plasma needed for the blood test. The width W of the micro space 114, though not limited in particular, may preferably about equal to the thickness D. In the way it is illustrated in Fig. 7, the width W of the micro space 114 is set to be substantially twice as large as the thickness.

At one end of the container 112 in the lengthwise direction, a blood filling port 120 for filling the blood A is formed, and a plasma collecting port 122 is formed toward the upper face of the container 112. Out of the (six) faces constituting the cuboidal micro space 114, that toward the blood filling port 120 will be referred to as the top face 114A, the opposite one as the bottom face 114B, that toward the plasma collecting port 122 as the upper face 114C and the opposite one as the lower face 114D. An injecting pipe 124 is linked to the blood filling port 120, and a sucking pipe 130 is linked to the plasma collecting port 122 via a collection channel member 128 in which a collection channel 128A equal to the plasma collecting port 122 in the opening size is formed. In this case, if it is intended to keep the width W of the micro space 114 as thin as the thickness D for instance, it can be linked to the sucking pipe 130 without having the collection channel member 128 in-between. However, to make the width W of the micro space 114 greater, having the collection channel member 128 in-between enables the plasma B (see Figs. 8A to 8C) having deposited above the upper face of the micro space 114 as a result of solid/liquid separation to be described afterwards to be sucked uniformly in the widthwise direction of the micro space 114.

Where the collection channel member 128 is to be disposed, it will not be acceptable for the blood A filling the micro space 114 to reach the collection channel 128A because this would elongate the effective precipitation distance of the corpuscles C. Therefore, such contrivances may be made, for instance, as reducing the thickness E of the collection channel 128A to enable the surface tension of the blood A to prevent the blood A from entering into the collection channel 128A or treating the inside surface of the collection channel 128A for hydrophobicity to prevent the blood A from entering into the collection channel 128A by repelling the blood A.

As the filling device 116 for filling the blood A into the micro space 114, for instance a syringe 116 can be suitably used as shown in Fig. 7. Thus, the needle 116A of the syringe 116 holding the blood A inside is inserted into the injecting pipe 124, and the blood in the syringe 116 is injected into the micro space 114 by pressing the piston 116B in.

As the sucking device 118 for collecting the plasma B, for instance a continuously fluid type syringe pump 118 also provided with a suction pressure control function can be suitably used as shown in Fig. 7. This enables the suction pressure and the suction flow rate to be controlled as desired. Thus, the tip outlet 118A of the syringe pump 118 is connected to the sucking pipe 130, and a collecting tube 132 is also connected to a blood testing apparatus (not shown). This enables the plasma (supernatant liquid) separated in the micro space 114 to be directly fed to the blood testing apparatus via the collection channel member 128.

As the inclining device 119 for inclining the container 112, what is shown in Figs. 8A to 8C can be used for instance. As shown in Figs. 8A to 8C, the inclining device 119 is configured by supporting at the center of the upper face of a base 119A a supporting member 119C via a rocking mechanism 119B to be rockable in the directions of arrow a-b. The supporting member 119C has a concave section, and the lower part of the container 112 is detachably snapped into concave. As the rocking mechanism 119B, any mechanism which can keep the container 112 supported by the supporting member 119C immovable from a prescribed angle of inclination can be used.

Micro machining techniques can be suitably applied to the fabrication of the container 112 having the micro space 114 and the collection channel member 128 having the collection channel 128A. Description of the micro machining techniques is dispensed with here because they are the same as those described with reference to the first embodiment.

Available materials for the container 112 and the collection channel member 128 include metals, glass, ceramics, plastics, silicon and resins such as Teflon^{®}, from which one or another can be selected according to different requirements regarding resistance to heat, resistance to pressure, resistance to solvents and machining ease among other factors, and particularly preferable ones include polystyrene resin, PMMA resin, quartz glass and Pyrex®. As will be described afterwards with reference to the plasma collecting method, it is preferable for the container 112 to be fabricated of a transparent material so that the state of separation between the plasma B and the corpuscles C can be visually recognized.

It is preferable for the container 112 to have a heating device (not shown). What was described regarding the heating device for the first embodiment applies here as well.

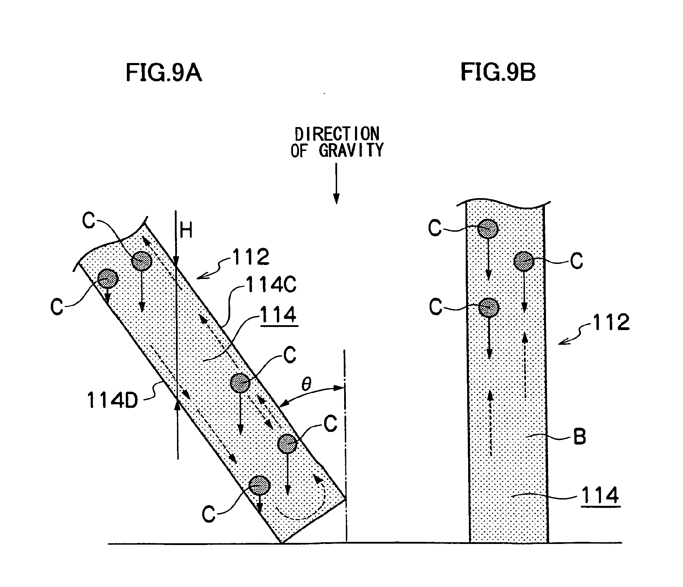

Next, the procedure of plasma collection according to the invention using the plasma collecting tool 100 configured as stated above will be described with reference to Fig. 7 to Figs. 9A and 9B.

The blood plasma collecting method according to the invention mainly comprises a filling step of filling with blood A the container 112 having the micro space 114 of an extremely thin rectangular shape measuring 50 µm to 2 mm in thickness, an inclining step of inclining the container 112 to an inclination angle of 30 to 60° to the vertical direction (the direction of gravity), a solid/liquid separation step of subjecting the blood A to solid/liquid separation by gravity into plasma B and corpuscles C, and a collection step of collecting the plasma B having undergone solid/liquid separation by sucking from the container 112.

First, as shown in Fig. 8A, the container 112 held by the inclining device 119 is stood at the filling step, and the syringe 116 shown in Fig. 7 is used to fill the micro space 114 in the container 112 with the blood A. In this case, the sucking pipe 130 is opened to the atmosphere to be used as an air escape.

Next, at the inclining step as shown in Fig. 8B, the container 112 is inclined to an inclination angle of 30 to 60°, preferably 45°, to the vertical direction (the direction of gravity). In this case, the container 112 is so inclined that its upper face (meaning the same as the upper face 114C of the micro space 114) in which the collection channel member 128 is disposed faces upward. The container 112 may be inclined either before or after the container 112 is filled with the blood A. Further at the filling step, the container 112 need not be filled in a standing state, but may be raised to an inclination angle of 30 to 60° after it is filled in a laid state.

Next, at the solid/liquid separation step illustrated in Fig. 8B and Fig. 8C, the Boycott effect is given rise to in the micro space 114 by implementing the solid/liquid separation step in an inclined state of the container 112. Thus, as shown in Fig. 9A, while the corpuscles C precipitate in the direction of gravity, the plasma B rise along the upper face 114C of the micro space 114 (the inner wall face toward the upper face of the container). As a result, in the micro space 114, a ring flow (represented by dotted arrows in Fig. 9A) of the plasma B is formed as an upward flow along the upper face 114C of the micro space 114 becomes a downward flow along the lower face 114D of the micro space 114. As this serves to prevent the precipitation of the corpuscles C and the upward flow of the plasma B from colliding head on, the plasma and the corpuscles can be separated in a short period of time. Furthermore, by inclining the container 112, the precipitating distance H of the corpuscles C can be significantly shortened as is seen from Fig. 9A. The corpuscles C having descended over the precipitating distance H and collided with the lower face 114D of the micro space ride on the ring flow of the plasma B to be carried toward the bottom face 114B of the micro space 114.

As a result, the plasma B and the corpuscles C can be accurately separated in a short period time from each other as shown in Fig. 8C. More specifically, by keeping the blood A at rest in the inclined micro space 114 for three to five minutes, the blood A can be subjected to solid/liquid separation by gravity into the plasma B and the corpuscles C. It is preferable at this solid/liquid separation step to keep the container 112 at 11 to 40°C to reduce the viscosity of the blood A, and this could further accelerate the precipitating velocity of the corpuscles C.

On the other hand, if the solid/liquid separation step is implemented in a state in which the container 112 is erected, the precipitation of the corpuscles C and the upward flow of the plasma B will collide head on to bring down the precipitating velocity of the corpuscles C significantly as shown in Fig. 9B. Moreover, the corpuscles C would then flow down over a precipitating distance corresponding to the length L of the micro space 114, the separation would take an extremely long time.

The Boycott effect is discussed in detail in the *Nature* magazine (Boycott, A.E., Sedimentation of Blood Corpuscles, Nature, London), 104.532., issued in 1920) and the Biorheology magazine (in Japanese, published by Yoneda Shuppan, an article by Shin Kaihara and Akio Sakanishi, first edition issued on June 4, 1999).

Upon completion of the solid/liquid separation step, the syringe pump 118 is connected to the sucking pipe 130 as shown in Fig. 7 to implement the collection step of collecting only the plasma B as the supernatant liquid. Thus, it is sufficient for the sucking force of the syringe pump 118 to be able to break the surface tension of the plasma B on the boundary between the micro space 114 and the collection channel 128A and to break the force of the collection channel 128A by its hydrophobicity to prevent the plasma from flowing in. It is preferable, for instance, for the sucking force to exert itself to so cause the plasma B in the micro space 114 as to flow into the collection channel 128A at a rate of not more than 1 mm/second. Too great a sucking force might whirl up the corpuscles C which have precipitated to become mixed with the plasma B to be collected. The collected plasma B is fed to the blood testing apparatus via the collecting tube 132 of the syringe pump 118.

By utilizing the Boycott effect in the micro space 114 in this way, the plasma B and the corpuscles C can be separated from each other with a small quantity of blood in an extremely short period of time. This enables the plasma B and the corpuscles C to be separated from each other requiring only a small quantity of blood in a short period of time easily and yet accurately with a small, simple-structured tool to enable the plasma B to be collected without needing the use of any chemical such as a separation assisting agent.

### (Simplified Blood Testing Method and Tool)

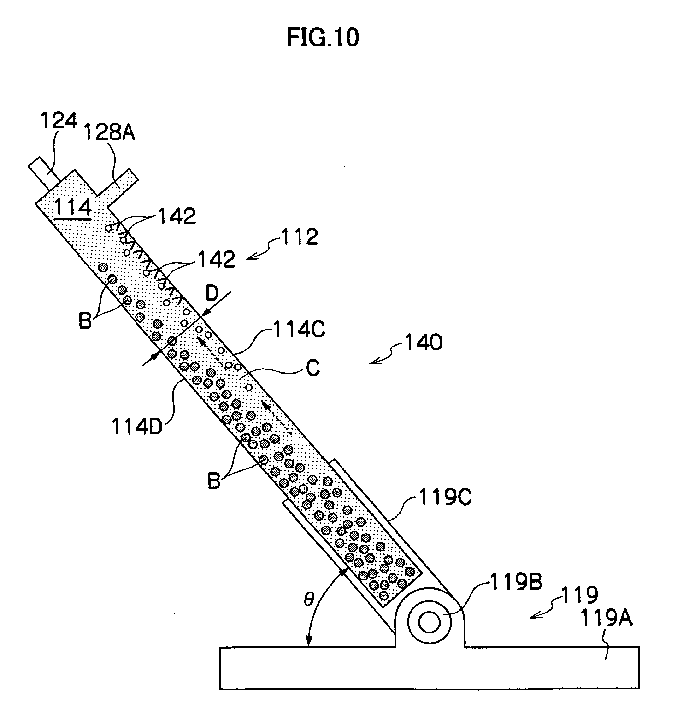

Fig. 10 is a conceptual diagram of one example of simplified blood testing tool 140 according to the invention.

As shown in Fig. 10, the configuration of the simplified blood testing tool 140, though basically similar to that of the plasma collecting tool 100, differs in that a testing reagent 142 is fastened to the upper part of the upper face 114C of the micro space 114 in advance. Incidentally, since the probability of the presence of the corpuscles C in the plasma B is higher in the lower part of the upper face 114C of the micro space 114, it is preferable to fasten the testing reagent 142 only to the upper part of the upper face 114C of the micro space 114, but the testing reagent 142 may as well be fastened substantially all over the upper face 114C.

The external shape of the simplified blood testing tool 140 also is basically similar to the plasma collecting tool 100 shown in Fig. 7, and the only differences are that the sucking device 118 in Fig. 7 is absent and the collection channel 128A and the sucking pipe 130 are used as air escapes.

As the testing reagent 142, an antibody can be suitably used. Testing by a so-called enzyme-linked immunosorbent assay (ELISA) can be performed by causing the antibody fastened to the upper part of the upper face 114C of the micro space 114 to react to any antigen in the plasma B.

A sandwich method, one version of ELISA, which is a simple testing method by which the antibody to any antigen in the test object is fixed and an antigen-antibody reaction is performed by using a second antibody labeled with fluorescence, a pigment, a radioactive substance, an enzyme or the like. Since the antigen need not be directly marked, reaction can be easily detected with high sensitivity. As the testing reagent 142, an antibody is not the only choice, but any reagent which reacts with a content in the plasma to allow some test or another can be used.

A suitable method of fastening the testing reagent 142 to the upper part of the upper face 114C of the micro space 114 is to spray the testing reagent 142 ejected from the nozzle of an ink jet device, for instance, onto to the upper part of the upper face 114C of the micro space 114, but the suitable method is not limited to this.

Next, the procedure of the simplified blood testing method using the simplified blood testing tool 140 configured as stated above will be described.

The sequence from the blood A filling step to the solid/liquid separation step is the same as in the plasma collecting method described above. In parallel with this solid/liquid separation step, a testing step is implemented. Thus, as already stated with reference to Fig. 9A, the upward flow of the plasma B is formed in the micro space 114. This enables the plasma B and the corpuscles C to be separated from each other without inviting collision between the precipitation of the corpuscles C and the upward flow of the plasma B and at the same time, as shown in Fig. 10, the upward stream of the plasma B (represented by dotted arrows in Fig. 10) flows along the upper face 114C of the micro space 114 and reacts with the testing reagent 142 fastened to the upper part of the upper face 114C with which it comes into efficient contact.

By applying the Boycott effect to the micro space 114 in this way, the upward stream of the plasma B is caused to flow along the upper face 114C of the micro space 114, enabling the plasma B to exert its stirring effect and providing an effect of dramatically enhancing the probability for a content in the plasma B to come into contact with the upper face of the micro space 114. Therefore, by fastening the testing reagent 142 to the upper part of the upper face 114C of the micro space 114 in advance, the efficiency of the substance to be tested in (a content of) the plasma B to come into contact with the testing reagent 142 is enhanced, and accordingly the sequence from plasma separation to blood testing can be accomplished in a consistent accurate process in a simple space such as the micro space 114.

### [Implementation Examples]

### [Implementation Example 1]

Next, Implementation Example 1 of the first preferred embodiment of the invention using the plasma collecting tool 10 shown in Fig. 1 will be described.

As the micro space 14 formed in the transparent tool body 12, what had a section measuring 400 µm in width W and 200 µm in depth D and a length L of 10 cm was used. The collection channel 20 that was used measured 100 µm in thickness D', 200 µm in width W' and 2 cm in length L'.

Then, the blood A (50% in hematocrit value) was introduced into the micro space 14 with the syringe 16. A sufficient quantity of the blood A was put into to fill the space up to the boundary 42 between the micro space 14 and the collection channel 20. Incidentally, 3.8% sodium citrate had been mixed in advance with the blood A that was used in a volume ratio of 4 (blood):1 (sodium citrate) to prevent coagulation. The quantity of the blood A that filled the micro space 14 was about 8 µL.

After that, the blood A was allowed to stand still in the micro space 14 to subject the plasma B and the corpuscles C in the blood A to solid/liquid separation. The time taken by the plasma B and the corpuscles C to be separated from each other was measured, and this proved to be as short as about three and a half minutes. To collect only the separated plasma B, suction was preformed with the syringe pump 18. The rate of flow of the plasma B by suction was set to 5 µL per minute (flow rate = 1 mm /minute). As a result of continued suction for about one minute without allowing corpuscles to accompany, the collected quantity of the plasma reached about 1 µL.

In this way, by using the plasma collecting tool 10 according to the invention, the plasma and the corpuscles can be separated from each other requiring only a small quantity of blood in a short period of time easily and yet accurately with a small, simple-structured tool to enable the plasma to be collected without needing the use of any chemical such as a separation assisting agent.

In applying this blood plasma collecting method, it is preferable not to collect the whole quantity of the separated plasma B. The reason is that, when the collection of the plasma B proceeds to replace the plasma B in the micro space 14 with air, the corpuscles C will be significantly whirled up on the air-liquid interface along with their transfer due to suction. More specifically, it is preferable to collect not more than 1/3 of the quantity of plasma separated in the micro space 14. Therefore, it is advisable to take into account the quantity of plasma to be collected and that of plasma required for the blood test in determining the capacity of the micro space 14 (whose depth is fixed to between 10 and 1000 µm, though).

### [Implementation Example 2]

As another specific example of the implementation of the invention, the simplified blood testing method by testing the antigen in the plasma B by ELISA with the simplified blood testing tool 140 shown in Fig. 10 will be described.

The micro space 114 having a section of 200 µm in thickness and 400 µm in width and a length L of 2 cm was formed in the container 112 fabricated of polystyrene. Anti-human IgG mouse antibody was fastened as the primary antibody to the upper part of the upper face 114C of the micro space 114 in advance. The fastening method was spraying of the primary antibody ejected from the ink ejection nozzle of an ink jet device onto the upper part of the upper face 114C of the micro space 114.

Next, a BSA buffer for restraining non-specific adsorption (phosphoric acid-buffered physiological saline solution with 1% fetal bovine serum added) was introduced into the micro space 114, and kept reacting for 10 minutes at 37°C to cover with BSA other parts than the surface to which the primary antibody was fastened. Afterwards, after washing that micro space 114 twice with a phosphoric acid buffer (phosphoric acid-buffered physiological saline solution), the micro space 114 was filled with 1.5 µL of blood (50% in hematocrit value).

Next, as shown in Fig. 10, the container 112 was allowed to stand still with an inclination of 45 degrees to the vertical direction (the direction of gravity). Incidentally, 3.8% sodium citrate had been mixed in advance with the blood A that was used in a volume ratio of 4 (blood) :1 (sodium citrate) to prevent coagulation. Further, this blood was kept in reaction with peroxidase-labeled anti-human immunoglobulin antibody as the labeling second antibody for five minutes at room temperature as advance reaction of the IgG antigen and the labeled secondary antibody.

As a result, the corpuscles C gradually precipitated in the micro space 114 and, in three minutes after the container 112 was inclined, a plasma phase, which was the phase of the transparent plasma B, and a blood corpuscle phase, which was an aggregate of the corpuscles C, began to emerge definitely. At this stage, the IgG having combined with the labeling second antibody in the plasma was reacted with the primary antibody and fixed to the surface.

Next, the inside of the micro space 114 was washed with a washing buffer (phosphoric acid-buffered physiological saline solution with 0.05% Tween 20 added), and the whole amount of blood that had once filled the micro space 114 was washed out. Then, as much as possible of the corpuscles was also washed out to minimize, if not eliminate, the residue.

Next, 1.5 µL of a substrate for color development (prepared by dissolving 2, 2'-azino-bis[3-ethylbenzothiazoline-6-sulfonate] (ABTS) in a mixed citric acid/phosphoric acid buffer (a solution prepared by adding an aqueous solution of 0.05M sodium hydrogen phosphate to an aqueous solution of 0.05 M citric acid until pH reached 4.0) to achieve a concentration of 0.7 mg/mL) was added into the micro space 114. The mixture was intercepted from light during the reaction. ABTS manifested a bluish green color by its reaction with peroxidase, a label enzyme. This reaction was continued for 10 minutes and, after stopping the reaction with reaction stopper liquid (0.01 % sodium azide liquid), the upper part of the upper face 114C of the micro space 114, the area where the plasma had deposited, was measured with an ELISA detecting device at a light emitting intensity of 405 nm.

Reading of the result from a working curve prepared in advance (the relationship between the light emitting intensity and the IgG quantity) resulted in the detection of 1 ng/mL of IgG at the maximum. It is necessary that the region where the light emitting intensity was measured should be the same as the region where the working curve was prepared. Further, the background intensity was measured with the micro space 114 filled with a mixed citric acid/phosphoric acid buffer containing no ABTS.

As hitherto described, the use of the simplified blood testing tool 140 according to the invention enables the sequence from plasma separation to blood testing to be accurately accomplished in a very small space such as the micro space 114 in a process comprising only a few steps.

## Claims

1. A blood plasma collecting method to separate corpuscles from blood and collect plasma, comprising:
a filling step of filling a micro space (14) which is at least one long space formed in the horizontal direction, measuring (10) µm to 1 mm in depth in the direction of gravity, with blood;
a solid/liquid separation step of subjecting the blood to solid/liquid separation by gravity into plasma and corpuscles by allowing the blood to stand still in the micro space (14) for a prescribed length of time; and
a collection step of collecting the plasma, which is the supernatant liquid resulting from the solid/liquid separation, by sucking a collection channel (20) which communicates with the micro space (14) upward in the direction of gravity and is smaller than the micro space (14) in equivalent diameter.

2. The blood plasma collecting method according to Claim 1, wherein at the filling step:
blood is injected into a liquid reservoir (22) formed at one lengthwise end of the micro space (14); and
blood in the liquid reservoir (22) is so filled into the micro space (14) as not to reach the collection channel (20) by sucking air in the micro space (14) through an air escape (24) formed at the other lengthwise end of the micro space (14) or pressurizing the liquid reservoir (22) in a state in which the collection channel (20) is sealed or pressurized.

3. The blood plasma collecting method according to Claim 2, wherein the micro space (14) is treated for hydrophilicity and the collection channel (20) is treated for hydrophobicity.

4. The blood plasma collecting method according to any one of Claims 1 through 3, wherein at the solid/liquid separation step the temperature of the blood is kept in a range of 11 to 40°C.

5. The blood plasma collecting method according to any one of Claims 1 through 4, wherein at the solid/liquid separation step the prescribed length of time for allowing the blood to stand still is within a range of three to six minutes.

6. The blood plasma collecting method according to any one of Claims 1 through 5, wherein at the collection step plasma is sucked in a state in which the collection channel (20) communicates with elsewhere than any lengthwise end of the micro space (14).

7. The blood plasma collecting method according to any one of Claims 1 through 6, wherein at the collection step the plasma as the supernatant liquid of the micro space (14) is so sucked into the collection channel (20) at a flow rate of 1 mm/second or below.

8. A blood plasma collecting tool (10) to collect separate corpuscles from blood and collect plasma, comprising:
a tool body (12) within whose substrate a micro space (14) which is at least one long space formed in the horizontal direction, measuring 10 µm to 1 mm in depth in the direction of gravity, a collection channel (20) which communicates with the micro space (14) upward in the direction of gravity and is smaller than the micro space (14) in equivalent diameter, a liquid reservoir (22) which, formed at one lengthwise end of the micro space (14), communicates with the atmosphere, an air escape (24) which, formed at the other lengthwise end of the micro space (14), communicates with the atmosphere, and a sucking part (26) which, formed at the tip of the collection channel (20), communicates with the atmosphere are formed;
a filling device (16) which fills the inside of the micro space (14) with blood via the liquid reservoir (22); and
a sucking device (18) connected to the sucking part (26) .

9. The blood plasma collecting tool (10) according to Claim 8, wherein the collection channel (20) is connected to the central position lengthwise of the micro space (14).

10. The blood plasma collecting tool (10) according to Claim 8 or 9, wherein the width of the long micro space (14) is in a range of 50 µm to 5 mm and the length of the same in the horizontal direction is from 1 to 20 cm.

11. The blood plasma collecting tool (10) according to any one of Claims 8 through 10, wherein the inside surface of the micro space (14) is treated for hydrophilicity and the inside surface of the collection channel (20) is treated for hydrophobicity.

12. The blood plasma collecting tool (10) according to any one of Claims 8 through 11, wherein the tool body (12) is transparent.

13. The blood plasma collecting tool (10) according to any one of Claims 8 through 12, wherein the material of the tool body (12) is any of polystyrene resin, acryl resin, PDMS resin, polycarbonate resin, PET resin, quartz glass, Pyrex® and silicon.

14. The blood plasma collecting tool (10) according to any one of Claims 8 through 13, wherein a plurality of the collection channels (20) are provided at intervals in the lengthwise direction of the micro space (14) .

15. A blood plasma collecting method to separate corpuscles from blood and collect plasma, comprising:
a filling step of filling with blood a container (112) having a micro space (114) of an extremely thin rectangular shape measuring 50 µm to 2 mm in thickness;
an inclining step of inclining the container (112) to an inclination angle of 30 to 60° to the vertical direction before or after the filling step;
a solid/liquid separation step of subjecting blood to solid/liquid separation by gravity into plasma and corpuscles by allowing the blood to stand still for a prescribed length of time in the inclined container (112) ; and
a collection step of collecting the plasma having undergone solid/liquid separation by sucking from the container (112) .

16. The blood plasma collecting method according to Claim 15, wherein at the solid/liquid separation step the temperature of the blood is kept in a range of 11 to 40°C.

17. The blood plasma collecting method according to Claim 15 or 16, wherein at the solid/liquid separation step the prescribed length of time for allowing the blood to stand still is within a range of three to five minutes.

18. A simplified blood testing method for testing blood in a simplified way, comprising:
a filling step of filling with blood a container (112) having a micro space (114) of an extremely thin rectangular shape measuring 50 µm to 2 mm in thickness;
an inclining step of inclining the container (112) to an inclination angle of 30 to 60° to the vertical direction before or after the filling step;
a solid/liquid separation step of subjecting blood in the inclined container (112) to solid/liquid separation by gravity into plasma and corpuscles by allowing the blood to stand still for a prescribed length of time; and
a testing step of bringing a substance to be tested in the plasma having undergone the solid/liquid separation into contact with a testing reagent (142) fastened in advance to the upper part of the upper face (114C) of the opposing faces of the micro space (114) in the thickness direction in the inclined state, and thereby conducting a test.

19. The simplified blood testing method according to Claim 18, wherein the substance to be tested in the plasma is an antigen, the testing reagent (142) is an antibody, and the testing is accomplished by causing an antigen-antibody reaction to take place at the testing step.

20. A plasma collecting tool (100) which separates corpuscles from blood and collects plasma, comprising:
a container (112) having inside a micro space (114) of an extremely thin rectangular shape measuring 50 µm to 2 mm in thickness, and a blood filling port (120) and a plasma collecting port (122) both communicating with the micro space (114) ;
a filling device (116) which fills the micro space (114) of the container (112) with blood via the blood filling port (120) ;
an inclining device (119) which inclines the container (112) to achieve a prescribed angle of inclination relative to the vertical direction; and
a sucking device (118) which collects plasma by sucking the same through the plasma collecting port (122).

21. The plasma collecting tool (100) according to Claim 20, wherein the angle of inclination is in a range of 30 to 60°.

22. The plasma collecting tool (100) according to Claim 20 or 21, wherein an aspect ratio represented by L/D, where D is the thickness and L is the length of the micro space (114), is within a range of 5 to 50.

23. The plasma collecting tool (100) according to any one of Claims 20 through 22, wherein the container (112) is transparent.

24. The plasma collecting tool (100) according to any one of Claims 20 through 23, wherein the material of the container (112) is any out of resins, quartz glass and Pyrex^{®}.

25. The plasma collecting tool (100) according to any one of Claims 20 through 24, further provided with a heating device for heating the container (112).

26. A simplified blood testing tool (140) for simplified testing of blood, comprising:
a container (112) having inside a micro space (114) of an extremely thin rectangular shape measuring 50 µm to 2 mm in thickness, wherein a testing reagent (142) is fastened in advance to one of the opposite faces of the micro space (114) opposing in the thickness direction, and a blood filling port (120) and an air escape both communicating with the micro space (114);
a filling device (116) which fills the micro space (114) of the container (112) with blood via the blood filling port (120); and
an inclining device (119) which inclines the container (112) to achieve a prescribed angle of inclination relative to the vertical direction and to position upward the one face to which the testing reagent (142) is fastened.

27. The simplified blood testing tool (140) according to Claim 26, wherein the testing reagent (142) is an antibody, and an antigen in the plasma, which is a content of the blood, is tested.
